(19) Europäisches Patentamt
European Patent Office
Office européen des brevets

(11) Numéro de publication : **0 419 647 B1**

# (12) FASCICULE DE BREVET EUROPEEN

(45) Date de publication du fascicule du brevet : **01.09.93 Bulletin 93/35**

(51) Int. Cl.$^5$ : **C07C 311/48**

(21) Numéro de dépôt : **90907110.2**

(22) Date de dépôt : **05.04.90**

(86) Numéro de dépôt international : **PCT/FR90/00240**

(87) Numéro de publication internationale : **WO 90/11999 18.10.90 Gazette 90/24**

## (54) PROCEDE DE SYNTHESE DE SULFONYLIMIDURES.

(30) Priorité : **06.04.89 FR 8904504**

(43) Date de publication de la demande : **03.04.91 Bulletin 91/14**

(45) Mention de la délivrance du brevet : **01.09.93 Bulletin 93/35**

(84) Etats contractants désignés : **AT BE CH DE DK ES FR GB IT LI LU NL SE**

(56) Documents cités :
**EP-A- 0 096 629**
**BE-A- 696 926**
**DE-A- 2 239 817**
**US-A- 4 505 997**

(56) Documents cités :
**CHEMICAL ABSTRACTS, volume 74, no. 9, 1er mars 1971,Columbus, Ohio, US; A.J. GORDON et al.: "Chemistry of imides. II. Cyclic imides and some chlorides and lithium nitride", voir page 312, abrégé 41848d, & J. Org. Chem. 1971, 36(1), 44-5**

(73) Titulaire : **CENTRE NATIONAL DE LA RECHERCHE SCIENTIFIQUE**
**15, quai Anatole France**
**F-75007 Paris (FR)**
Titulaire : **HYDRO-QUEBEC**
**75, Boulevard René Levesque Ouest**
**Montréal Québec H2Z 1A4 (CA)**

(72) Inventeur : **ARMAND, Michel**
**"Les Corjons"**
**F-38410 S.-Martin-d'Uriage (FR)**

(74) Mandataire : **Sueur, Yvette**
**Cabinet Yvette Sueur 78, rue Carnot**
**F-95240 Cormeilles-en-Parisis (FR)**

EP 0 419 647 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne un procédé de synthèse de sulfonylimidures et plus particulièrement de perfluorosulfonylimidures symétriques.

Les perfluorosulfonylimidures répondant à la formule générale $M[(R_FSO_2)_2N]_y$, dans laquelle M désigne un métal ou un groupement ammonium quaternaire ou non, les $R_F$, identiques dans le cas d'imidures symétriques ou différents dans le cas d'imidures dissymétriques, représentent des radicaux monovalents perfluorohydrocarbyles et notamment des radicaux perfluoroalcoyles tels que $CF_3$, $C_2F_5$, $C_4F_9$ ou des radicaux perfluoroaryles tels que $C_6F_5$, et y est un nombre égal à la valence de M, sont intéressants par les propriétés liées à l'anion correspondant. En effet, la délocalisation de la charge de l'anion sur plusieurs centres électronégatifs, à savoir les atomes F, O et N, induit une basicité et un caractère nucléophile faibles. La stabilité des liaisons covalentes permet de plus un domaine étendu de stabilité redox, en particulier aux potentiels anodiques. Les perfluorosulfonylimidures de métaux alcalins et notamment de lithium sont en particulier utilisables pour former des solutions solides avec des matériaux macromoléculaires du type des polyéthers, lesdites solutions solides trouvant une application comme électrolytes solides polymères dans la production de générateurs primaires ou secondaires tout-solide en films minces (US-A-4505997). Ils sont également utiles comme sels dans les électrolytes liquides.

L'un de ces procédés décrit dans EP-96629 consiste tout d'abord à faire réagir l'anhydride $(R_FSO_2)_2O$ avec l'urée et un acide sulfonique $R_FSO_3H$ selon la réaction :

$$(R_FSO_2)_2O + R_FSO_3H + OC(NH_2)_2 \text{ ----> } (R_FSO_2)_2NH\ NH_4CF_3SO_3 + CO_2\nearrow$$

Les produits obtenus après réaction sont ensuite dissous dans l'eau et l'addition de bromure de tétrabutylammonium à la solution obtenue permet de précipiter l'imidure de tétrabutylammonium de formule $(Bu)_4NN(R_FSO_2)_2$.

Par une réaction d'échange ionique entre ce composé et le tétraphénylborohydrure de sodium, on forme l'imidure de sodium $NaN(R_FSO_2)_2$.

Le second desdits procédés consiste à faire réagir l'anhydride $(R_FSO_2)_2O$ avec la pyridine et le chlorure d'ammonium selon le schéma réactionnel :

$$NH_4Cl + 2(R_FSO_2)_2O + 4C_5H_5N \text{ ----> } C_5H_5NH\ N(R_FSO_2)_2 + C_2H_5NHCl + 2(C_2H_5NH)CF_3SO_3$$

Les produits de la réaction sont dissous dans l'eau et l'addition de bromure de tétrabutylammonium à la solution obtenue conduit à la précipitation de l'imidure de tétrabutylammonium puis aux sels d'autres métaux comme indiqué pour la première méthode.

Les procédés précités ne sont pas satisfaisants pour une production des imidures à grande échelle car les rendements globaux sont faibles et les anhydrides précurseurs $(R_FSO_2)_2O$ ne sont pas accessibles aisément.

On peut encore obtenir les imidures mentionnés plus haut à partir des précurseurs $R_FSO_2F$ en faisant appel au procédé de synthèse en quatre étapes proposé par J. FOROPOULOS et D.D. DESMARTEAU dans la revue INORGANIC CHEMISTRY, Vol. 23 (1984), N° 23, pages 3720 à 3723. Dans ce procédé qui conduit à l'imidure de sodium, le schéma réactionnel est le suivant :

1)

$$R_FSO_2F + NH_4 \text{ --------> } NH_4NHSO_2R_F \xrightarrow{HCl} H_2NSO_2R_F$$

2) $H_2NSO_2R_F + NaOCH_3$ ------> $NaNHSO_2R_F + HOCH_3$

3) 2 $NaNHSO_2R_F + [(CH_3)_3Si]_2NH$ ----> $2(CH_3)_3Si\ NNaSO_2R_F + NH_3\nearrow$

4) $(CH_3)_3SiNNaSO_2R_F + R_FSO_2F$ ----> $NaN(SO_2R_F)_2 + (CH_3)_3SiF\nearrow$

Pour la quatrième étape de ce procédé, on se référera à DE 2 239 817.

Outre un rendement faible, généralement inférieur à 50 %, ce procédé ne peut se généraliser à l'utilisation de précurseurs du type chlorure de sulfonyle $R_FSO_2Cl$ car ces composés ne donnent pas, par action de l'ammoniac, l'amide $H_2NSO_2R_F$ qui est produite dans la première étape de la méthode et qui est nécessaire pour la mise en oeuvre des étapes ultérieures de cette méthode.

Un autre procédé consiste à obtenir des composés du type amide d'acide N-(amidosulfonyl)-sulfonique N'-substitué par réaction d'un amide d'acide sulfonique avec un halogénure d'acide sulfamique.

Un autre procédé a alors été mis au point pour la synthèse de sulfonylimidures de formule $M[(RSO_2)_2N]_y$, dans laquelle R représente un radical hydrocarbyle et plus particulièrement un radical perfluorohydrocarbyle $R_F$, et M et y ont les significations données plus haut, à partir des fluorures ou des chlorures de sulfonyle correspondants, utilisés comme précurseurs. Ce procédé consiste à faire réagir une composante silazane avec

une composante halogénure de sulfonyle. La composante silazane est un silazane ou l'association d'un dérivé silazane avec un fluorure ayant une faible énergie réticulaire. La composante halogénure de sulfonyle est un fluorure de sulfonyle ou l'association de chlorure de sulfonyle et d'un fluorure à faible énergie réticulaire. Ce procédé permet d'obtenir respectivement en une seule étape ou en deux étapes, les sulfonylimidures dissymétriques avec des rendements élevés. Toutefois les silazanes ou leurs dérivés sont d'un emploi délicat, car ils sont sensibles à l'air. En outre, il s'agit de composés onéreux.

On a maintenant trouvé un nouveau procédé de synthèse de sulfonylimidures à partir d'halogénure de sulfonyle et de nitrures ioniques, produits d'usage courant et d'un emploi aisé. Ce procédé est d'une grande simplicité à toute échelle et met en oeuvre les nitrures qui sont des composés de faible coût, d'emploi aisé (solides) et obtenus facilement par réaction directe des éléments (azote + métal).

L'invention a pour objet un procédé de synthèse de sulfonylimidures de formule générale $M[(RSO_2)_2N]_y$, dans laquelle M représente un métal choisi parmi les métaux alcalins, les métaux alcalino-terreux, les terres rares, Al, Sc, Y et Th, R représente un radical monovalent choisi parmi les radicaux aliphatiques ayant de 1 à 8 atomes de carbone linéaires ou ramifiés, les radicaux alicycliques ayant de 3 à 8 atomes de carbone, les radicaux aryliques ayant de 3 à 8 atomes de carbone et y est un nombre égal à la valence de M, caractérisé en ce que l'on fait réagir un nitrure ionique de formule $M_3N_y$ dans laquelle M et y ont la signification donnée ci-dessus, avec un halogénure de sulfonyle répondant à la formule $RSO_2X$, dans laquelle R a la signification donnée ci-dessus, et X est choisi parmi F ou Cl, dans un solvant aprotique polaire.

Les radicaux R peuvent comporter des atomes d'halogéne, par exemple des atomes de chlore ou de fluor.

Les radicaux R ayant de 1 à 4 atomes de carbone sont particulièrement intéressants.

Le procédé est mis en oeuvre à une température comprise entre 0°C et 150°C. Généralement une température inférieure à 40°C convient.

Le procédé est particulièrement adapté à la préparation de sulfonylimidures de métaux alcalins ou de métaux alcalino-terreux, en particulier de lithium et de magnésium. La réaction a lieu selon le schéma :

$$2yRSO_2X + M_3N_y \text{ -------->} 2yMX + M\,[(RSO_2)_2N]_y$$

Les nitrures ioniques appropriés pour le procédé de l'invention sont des composés qui contiennent l'entité $N^{3-}$ et qui s'hydrolysent facilement pour produire l'ammoniac. Comme exemples de tels nitrures, on peut citer les nitrures de métaux alcalins, de métaux alcalino-terreux, de terres rares, d'aluminium, de scandium, d'yttrium, de thorium.

Les nitrures préférés sont les nitrures de métaux alcalins et les nitrures de métaux alcalino-terreux.

Les solvants aprotiques polaires peuvent être choisis parmi les éthers tels que le tétrahydrofuranne (THF), le diméthoxyéthane (DME), les glymes, les amides tels que le diméthylformamide (DMF), la N-méthylpyrolidone (NMP), la tétraméthylurée (TMU), la diméthyléthylèneurée (DMU), la tétraéthylsulfonamide (TESA), le diméthylsulfoxyde (DMSO).

Lorsque l'halogénure de sulfonyle utilisé pour la réaction est un fluorure de sulfonyle, on utilise de préférence les éthers DME et THF qui forment des milieux suffisamment solvatants pour permettre une réaction rapide. Lorsque l'halogénure de sulfonyle utilisé est un chlorure, l'emploi de solvants très donneurs de doublets électroniques, du type amide, purs ou sous forme de mélanges avec des éthers, est nécessaire.

Le procédé de l'invention est particulièrement intéressant pour la synthèse d'imidures perfluorés, dans lesquels le radical R est un radical perfluoroalkyle ou perfluoroaryle.

La présente invention est décrite plus en détails par les exemples suivants, donnés à titre illustratif et non limitatif.

EXEMPLE 1

Préparation du bis trifluorométhanesulfonylimidure de lithium

A une suspension de 35 g de nitrure de lithium dans 500 ml de THF maintenue à - 18°C dans un autoclave, on a ajouté lentement 304 g de fluorure de trifluorométhanesulfonyle. Le récipient a été fermé et agité à la température de 50°C jusqu'à l'observation d'une chute de la pression dans le réacteur. La réaction s'est effectuée selon le schéma réactionnel suivant :

$$2CF_3SO_2F + Li_3N \text{ ------->} Li(CF_3SO_2)_2N + 2\text{<}LiF\text{>}$$

Le fluorure de lithium insoluble dans le THF a été éliminé par filtration ; le solvant a été évaporé et un résidu solide de bis trifluorométhanesulfonylimidure de lithium a été obtenu. Ce composé a été purifié par lavage au dichlorométhane et on a obtenu 260 g, correspondant à un rendement de 90 %.

EXEMPLE 2

Préparation du bis (perfluorobutanesulfonyl) imidure de lithium

6,04 g de fluorure de perfluorobutane sulfonyle ont été ajoutés à 350 mg de nitrure de lithium en suspension dans 20 ml de THF anhydre.
Le mélange a été agité à température ambiante pendant 48 h. La réaction s'est effectuée selon le schéma réactionnel suivant :

$$2C_4F_9SO_2F + Li_3N \text{ -------> } Li(C_4F_9SO_2)_2N + 2 <LiF>$$

L'imidure produit de la réaction a été obtenu par évaporation du solvant après filtration pour éliminer le florure de lithium insoluble formé. On a ainsi obtenu 4,9 g d'imidure de lithium, correspondant à un rendement de 84 %.

EXEMPLE 3

Préparation du bis (trifluorométhane sulfonyle) imidure de lithium

22 ml de chlorure de trifluorométhanesulfonyle ont été ajoutés à une suspension de 3,5 g de nitrure de lithium dans 100 ml d'un mélange de DMF et de DME (50/50). La dissolution du nitrure s'est produite en quelques minutes sous agitation à la température ambiante. La réaction s'est effectuée selon le schéma réactionnel suivant :

$$2CF_3SO_2Cl + Li_3N \text{ -------> } Li(CF_3SO_2)_2N + 2LiCl$$

La solution a été filtrée puis évaporée sous pression réduite. Le mélange de chlorure et d'imidure de lithium a été séparé par lavage à l'acétonitrile dans lequel seul l'imidure est soluble, et 26 g du sel de lithium du trifluorométhanesulfonimide ont été obtenus.
Le rendement en imidure était de : 90 %.

EXEMPLE 4

Préparation de bis (trifluorométhanesulfonyle) imidure de magnésium

A une suspension de 100 g de nitrure de magnésium en suspension dans 500 ml d'un mélange de dyglyme et de TMU (50/50) dans un autoclave et refroidie à - 20°C, on a ajouté 608 g de fluorure de trifluorométhanesulfonyle. Après fermeture de l'autoclave, le mélange a été porté à 80°C pendant 24 heures.
La réaction s'est effectuée selon le schéma réactionnel:

$$4CF_3SO_2F + Mg_3N_2 \text{ -------> } Mg[(CF_3SO_2)_2N]_2 + 2MgF_2$$

La solution obtenue a été filtrée, puis évaporée sous pression réduite.
On a alors obtenu un résidu sec constitué par 530 g d'imidure de magnésium correspondant à un rendement de 90,7 %.

EXEMPLE 5

Préparation du bis (méthanesulfonyl) imidure de lithium

A 23 g de chlorure de méthane sulfonyle $CH_3SO_2Cl$ dans 100 ml de DMF, on a ajouté 3,5 g de nitrure de lithium. Après dissolution de la phase solide, on a évaporé le solvant et on a lavé le résidu sec par du THF anhydre pour éliminer le chlorure de lithium formé. Le résidu sec a été extrait par le méthanol, puis desséché. On a obtenu 14,6 g de $Li(CH_3SO_2)_2N$ correspondant à un rendement de 82 %, selon le schéma réactionnel :

$$2CH_3SO_2Cl + Li_3N \text{ --------> } 2\ LiCl + Li(CH_3SO_2)_2N$$

Les sels obtenus par mise en oeuvre directe du procédé selon l'invention sont des produits stables et isolables. A partir de ces sels, on peut obtenir aisément les imides correspondants. Une possibilité consiste à acidifier une solution aqueuse du sel par un acide fort, puis à extraire l'imide formé par un solvant immiscible à l'eau, en particulier l'éther éthylique.

EXEMPLE 6

Préparation du bis (trifluorométhanesulfonyl) imide

Le résidu sec d'imidure de magnésium obtenu à l'exemple 4 a été traité par 200 ml d'acide sulfurique anhydre et distillé sous pression réduite (2 x $10^{-3}$ torr à 90°C). On a obtenu 500 g d'imide pure sous forme d'un solide hygroscopique fondant à 30 - 35°C.

Pour préparer un nouveau sel à partir de l'imide ainsi obtenu, on fait réagir l'imide avec un oxyde, un hydroxyde ou un carbonate approprié.

Ainsi, à partir des sulfonylimidures aisément accessibles par le procédé selon l'invention, on peut préparer, par simple échange de cations, des sulfonylimidures non accessibles par le procédé, par exemple parce que le nitrure correspondant n'existe pas. Parmi ces sulfonylimidures, on peut citer les sulfonylimidures d'ammonium quaternaire, et notamment les sulfonylimidures de tétra n-butylammonium.

Ces composés, plus particulièrement les trifluorométhane sulfonylimidures, sont particulièrement intéressants en électrochimie.

EXEMPLE 7

Préparation d'un film mince polymère - imidure

2,9 g du composé $Li(CF_3SO_2)_2N$ de l'exemple 1 ont été dissous avec 4,4 g de poly(oxyde d'éthylène) de masse moléculaire $5.10^6$ dans 200 ml d'acétonitrile. 15 ml de la solution visqueuse obtenue ont été coulés sur une plaque de polytétrafluoroéthylène dans un anneau de verre de 60 mm de diamètre. Après évaporation du solvant dans une étuve à 60°C, on a obtenu un film élastique et amorphe de 220 µm d'épaisseur du complexe polymère-sel. Ce matériau présente à 25°C une conductivité ionique de $2.10^{-5}$ (Ωcm) et peut être utilisé pour la constitution de générateurs primaires ou secondaires tout-solide dont l'électrode négative est constituée de lithium métallique ou d'un de ses alliages tel le lithiumaluminium.

## Revendications

1. Procédé de synthèse de sulfonylimidures de formule générale $M[(RSO_2)_2N]_y$, dans laquelle M représente un métal choisi parmi les métaux alcalins, les métaux alcalino-terreux, les terres rares, Al, Sc, Y et Th, R représente un radical monovalent choisi parmi les radicaux aliphatiques ayant de 1 à 8 atomes de carbone, linéaires ou ramifiés, les radicaux alicycliques ayant de 3 à 8 atomes de carbone, les radicaux aryliques ayant de 3 à 8 atomes de carbone et y est un nombre égal à la valence de M, caractérisé en ce que l'on fait réagir un nitrure ionique de formule $M_3N_y$ dans laquelle M et y ont la signification donnée ci-dessus, avec un halogénure de sulfonyle répondant à la formule $RSO_2X$, dans laquelle R a la signification donnée ci-dessus, et X est choisi parmi F ou Cl, dans un solvant aprotique polaire.

2. Procédé selon la revendication 1, caractérisé en ce que le radical R est chloré ou fluoré.

3. Procédé selon la revendication 2, caractérisé en ce que R est un radical perfluoroaryle.

4. Procédé selon l'une quelconque des revendications 1 à 2, caractérisé en ce que le radical R comporte de 1 à 4 atomes de carbone.

5. Procédé selon la revendication 4, caractérisé en ce que R est un radical perfluoroalkyle.

6. Procédé selon l'une quelconque des revendications 1 à 5, caractérisé en ce que M est choisi parmi les métaux alcalins et les métaux alcalino-terreux.

7. Procédé selon l'une quelconque des revendications 1 à 6, caractérisé en ce que le solvant polaire aprotique est choisi parmi les éthers, les amides, le diméthylsulfoxyde.

## Patentansprüche

1. Verfahren zur Synthese von Sulfonylimiden der allgemeinen Formel $M[(RSO_2)_2N]_y$, worin M ein Metall darstellt, das aus den Alkalimetallen, den Erdalkalimetallen, den seltenen Erden, Al, Sc, Y und Th ausgewählt ist, R einen einwertigen Rest darstellt, der aus den linearen oder verzweigten aliphatischen Resten mit 1 bis 8 C-Atomen, den alizyklischen Resten mit 3 bis 8 C-Atomen, und den Arylresten mit 3 bis 8 C-Atomen ausgewählt ist, und worin y eine Zahl gleich der Wertigkeit von M ist, dadurch gekennzeichnet, daß ein ionisches Nitrid der Formel $M_3N_y$, worin M und y die oben angegebene Bedeutung haben, mit einem Sulfonylhalogenid der Formel $RSO_2X$, worin R die oben angegebene Bedeutung hat und X aus F oder Cl ausgewählt ist, in einem aprotischen polaren Lösungsmittel umgesetzt wird.

2. Verfahren nach Anspruch 1, dadurch gekennzeichnet, daß der Rest R chloriert oder fluoriert ist.

3. Verfahren nach Anspruch 2, dadurch gekennzeichnet, daß R ein Perfluorarylrest ist.

4. Verfahren nach einem der Ansprüche 1 oder 2, dadurch gekennzeichnet, daß der Rest R 1 bis 4 C-Atome umfaßt.

5. Verfahren nach Anspruch 4, dadurch gekennzeichnet, daß R ein Perfluoralkylrest ist.

6. Verfahren nach einem der Ansprüche 1 bis 5, dadurch gekennzeichnet, daß M aus den Alkalimetallen und den Erdalkalimetallen ausgewählt ist.

7. Verfahren nach einem der Ansprüche 1 bis 6, dadurch gekennzeichnet, daß das aprotische polare Lösungsmittel aus den Äthern, den Amiden, und dem Dimethylsulfoxid ausgewählt ist.

## Claims

1. Process for the synthesis of sulfonylimides of the general formula $M[(RSO_2)_2N]_y$, in which M represents a metal chosen from alkali metals, alkaline earth metals, rare earths, Al, Sc, Y et Th, R represents a monovalent radical chosen from linear or branched aliphatic radicals having from 1 to 8 carbon atoms, alicyclic radicals having from 3 to 8 carbon atoms, aryl radicals having from 3 to 8 carbon atoms, and y is a number equal to the valence of M, characterized in that an ionic nitride of the formula $M_3N_y$, in which M and y have the meaning given above, is reacted with a sulfonyl halide of the formula $RSO_2X$, in which R has the meaning given above, and X is chosen from F or Cl, in an aprotic polar solvent.

2. Process according to claim 1, characterized in that the radical R is chlorinated or fluorinated.

3. Process according to claim 2, characterized in that R is a perfluoroaryl radical.

4. Process according to any one of claims 1 or 2, characterized in that the radical R contains 1 to 4 carbon atoms.

5. Process according to claim 4, characterized in that R is a perfluoroalkyl radical.

6. Process according to any one of claims 1 to 5, characterized in that M is chosen from alkali metals and alkaline earth metals.

7. Process according to any one of claims 1 to 6, characterized in that the aprotic polar solvent is chosen from ethers, amides and dimethylsulfoxide.